(19) Europäisches Patentamt
European Patent Office
Office européen des brevets

(11) Veröffentlichungsnummer: **0 020 858**
**A1**

(12) # EUROPÄISCHE PATENTANMELDUNG

(21) Anmeldenummer: **80101322.8**

(22) Anmeldetag: **29.06.78**

(51) Int. Cl.³: **C 07 D 283/02,** C 07 D 417/06, A 61 K 31/425, A 61 K 31/54

(30) Priorität: **07.07.77 LU 77703**

(43) Veröffentlichungstag der Anmeldung: **07.01.81 Patentblatt 81/1**

(84) Benannte Vertragsstaaten: **BE CH DE FR GB NL SE**

(71) Anmelder: **CIBA-GEIGY AG, Patentabteilung Postfach, CH-4002 Basel (CH)**

(72) Erfinder: **Göschke, Richard, Dr., Felixhäglistrasse 21, CH-4103 Bottmingen (CH)**
Erfinder: **Ferrini, Pier Giorgio, Dr., Im Rehwechsel 22, CH-4102 Binningen (CH)**

(74) Vertreter: **Zumstein sen., Fritz, Dr. et al, Bräuhausstrasse 4, D-8000 München 2 (DE)**

(54) **Cyclische Thiaaza-Verbindungen, Verfahren zu ihrer Herstellung, diese enthaltende pharmazeutische Präparate und ihre Verwendung.**

(57) Die Erfindung betrifft neue Thiaaza-verbindungen, insbesondere 1-Thia-3-azacyclopentane der allgemeinen Formel (I)

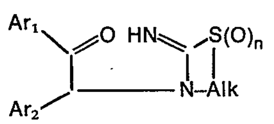

(I),

worin Alk Niederalkylen darstellt, welches das Thia- vom Aza-atom durch 2–4 Kohlenstoffatome trennt, $Ar_1$ und $Ar_2$ unabhängig voneinander gegebenenfalls substituiertes Phenyl, Pyridyl oder Thienyl bedeuten und n 0, 1 oder 2 ist, und deren Salze, sowie Verfahren zu ihrer Herstellung, ferner pharmazeutische Präparate enthalten diese Verbindungen und ihre Verwendung, vorzugsweise in Form von pharmazeutischen Präparaten.

Die neuen Verbindungen besitzen wertvolle pharmakologische Eigenschaften, insbesondere entzündungshemmende und antirheumatische Wirkung.

EP 0 020 858 A1

0020858

CIBA-GEIGY AG
Basel (Schweiz)

4-11226/+/B
Europa

BEZEICHNUNG GEÄNDERT
siehe Titelseite

Neue bicyclische Thiaaza-verbindungen und Verfahren zu deren Herstellung

Die Erfindung betrifft neue Thiaaza-verbindungen, insbesondere 1-Thia-3-aza-cyclopentane der allgemeinen Formel

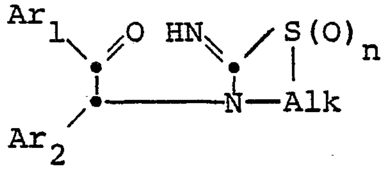

(I),

worin Alk Niederalkylen darstellt, welches das Thia- vom Aza-atom durch 2-4 Kohlenstoffatome trennt, $Ar_1$ und $Ar_2$ unabhängig voneinander gegebenenfalls substituiertes Phenyl, Pyridyl oder Thienyl bedeuten und n 0, 1 oder 2 ist, und deren Tautomere und Tautomeren Salze, sowie Verfahren zu ihrer Herstellung, ferner pharmazeutische Präparate enthaltend diese Verbindungen und ihre Verwendung, vorzugsweise in Form von pharmazeutischen Präparaten.

Die im Zusammenhang mit der vorliegenden Beschreibung mit "nieder" bezeichneten Reste und Verbindungen enthalten vorzugsweise bis 7 und in erster Linie bis 4 Kohlenstoffatome.

Niederalkylen Alk ist vorzugsweise unverzweigtes, aber auch verzweigtes Niederalkylen mit 2-4 Kohlenstoffatomen in der Kette zwischen dem Schwefel- und dem Stickstoffatom.

Pyridyl ist ein 2-, 3- oder 4-Pyridyl und Thienyl ein 3- oder insbesondere 2-Thienyl.

Substituiertes Phenyl, Pyridyl oder Thienyl ist z.B. einfach, zweifach oder auch mehrfach substituiert. Substituenten, insbesondere am Phenylrest sind u.a. Niederalkyl, Niederalkoxy, Halogen, Trifluormethyl, Niederalkylsulfonyl oder Nitro. Substituenten am Pyridyl- oder Thienyl-rest sind vorzugsweise Niederalkyl, Halogen oder Trifluormethyl.

Vorstehend wie nachfolgend können die Allgemeinbegriffe folgende Bedeutung haben:

Niederalkyl ist z.B. Methyl, Aethyl, n-Propyl, Isopropyl, n-Butyl, Isobutyl, sec.-Butyl oder tert.-Butyl, ferner n-Pentyl, n-Hexyl, Isohexyl oder n-Heptyl.

Niederalkylen ist Aethylen, sowie 1,3-Propylen, 1,4-Butylen, kann aber auch 1,2-Propylen, 1,2- oder 2,3-Butylen, 1,3- oder 2,4-Pentylen oder 1,4-Pentylen sein.

Niederalkoxy ist z.B. Methoxy, Aethoxy, n-Propyloxy, Isopropyloxy, n-Butyloxy oder tert.-Butyloxy.

Halogen ist solches mit der Atomnummer bis und mit 35 und steht für Fluor oder Brom, vorzugsweise für Chlor.

Niederalkylsulfonyl steht z.B. für Methylsulfonyl, Aethylsulfonyl oder n-Propylsulfonyl.

Die erfindungsgemässen Verbindungen besitzen wertvolle pharmakologische Eigenschaften, insbesondere entzündungshemmende und antirheumatische Wirkungen, wie sich in Tierversuchen zeigen lässt. z.B. im Kaolin-Pfotenoedem-Test (Helv. Physiol. Acta 25 (1967) 156) an der Ratte bei einer peroral gegebenen Dosis ab etwa 10 mg/kg oder im Terpentin-Pleuritis-Test [Helv. Physiol. Acta 26 (1969) 287] an der Ratte peroral gegeben bei einer Dosis von 30 bis 100 mg/kg zeigen sie eine antiinflammatorische bzw. anti-exsudative Wirkung. Sie zeigen

auch im Adjuvans-Arthritis-Test [Pharmacology 2 (1969) 288] an der Ratte bei einer peroralen Dosis von 10-30 mg/kg eine ausgezeichnete Wirkung.

Die neuen Verbindungen sind auch analgetisch wirksam, wie sich im Phenyl-p-benzochinon-Test an der Maus (Proc. Soc. Exp. Biol. 95 (1957) 729) bei Dosen von 30 bis 100 mg/kg, peroral gegeben, zeigen lässt.

Ferner ist die Hemmwirkung der neuen Präparate auf die Prostaglandin-Synthetase in vitro [Prostaglandins, 7 (1974) 123] in Konzentrationen von 0.05-20 µg/ml zu nennen. Ausserdem zeigen sie einen wertvollen antithrombotischen Effekt, nämlich einen Schutz vor tödlicher Lungenembolie am Kaninchen [Pharmacology 14 (1976) 522] in peroralen Dosen von 0.03-3 mg/kg.

Die neuen Verbindungen können desshalb als Antiphlogistika z.B. zur Behandlung von rheumatischen, arthritischen und anderen, mit Entzündungen verbundenen Erkrankungen insbesondere, rheumatischer Arthritis oder als Analgetika, z.B. zur Behandlung von Schmerzzuständen, verwendet werden.

Die Erfindung betrifft insbesondere Verbindungen der Formel I, worin $Ar_1$ und $Ar_2$ unabhängig voneinander einen gegebenenfalls durch Niederalkyl, Niederalkoxy, Halogen oder Trifluormethyl substituierten Phenylrest, einen Pyridyl-, wie einen 2-, 3- oder 4-Pyridyl-, oder Thienyl-, insbesondere einen 2-Thienylrest darstellen, Alk einen Niederalkylenrest, der das Schwefel- und Stickstoffatom über 2-3 Kohlenstoffatome miteinander verbindet, in erster Linie einen unverzweigten Niederalkylenrest und n besonders 0, ferner auch 1 oder 2 bedeuten, und deren Salze.

Die Erfindung betrifft besonders Verbindungen der der Formel II

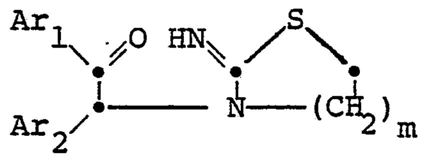

(II)

worin $Ar_1$ und $Ar_2$ unabhängig voneinander einen gegebenenfalls durch Niederalkoxy, wie Methoxy oder Halogen, insbesondere Chlor substituierten Phenylrest bedeuten und m in erster Linie 1, ferner auch 2 ist, und ihre Salze.

Insbesondere betrifft die Erfindung die neuen in den Beispielen beschriebenen Verbindungen.

Die neuen Verbindungen lassen sich nach an sich bekannten Methoden gewinnen, z.B. indem man eine Verbindung der Formel III

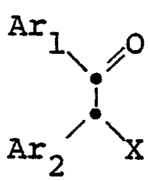

(III)

worin X eine reaktionsfähige veresterte Hydroxygruppe darstellt, mit einer Verbindung der Formel IV

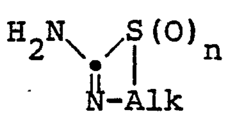

(IV)

oder einem Tautomeren und/oder Salz davon umsetzt, und, wenn erwünscht, in gegebenenfalls erhaltenen Verbindungen, worin n O ist, das Thiaatom zur Sulfinyl- oder Sulfonylgruppe oxidiert, und/oder, wenn erwünscht, erhaltene freie Verbindungen in ihre Salze überführt oder erhaltene Salze in die freien Verbindungen umwandelt und/oder ein verfahrensmässig erhaltenes Isomerengemisch in die einzelnen Isomeren trennt.

Reaktionsfähig verestertes Hydroxy ist insbesondere eine mit einer starken anorganischen Säure, z.B. Halogenwasserstoff, insbesondere Chlorwasserstoff, oder Schwefelsäure, oder mit einer starken organischen Säure, wie mit einer Niederalkansulfonsäure, z.B. Methan- oder Aethansulfonsäure oder einer gegebenenfalls durch Niederalkyl, Niederalkoxy oder Halogen substituierten Benzolsulfonsäure, z.B. p-Toluolsulfonsäure oder p-Brombenzolsulfonsäure veresterte Hydroxygruppe.

Die Umsetzung erfolgt unter wasserabspaltenden Bedingungen, wie durch Erwärmen, z.B. von etwa 50° bis etwa 150°, vorzugsweise in Gegenwart eines Lösungsmittels, wie Acetonitril oder einem Alkohol, z.B. Methanol oder Aethanol.

Die Oxydation des Thia-atoms zur Sulfinyl- oder Sulfonylgruppe lässt sich in an sich bekannter Weise z.B. mit Peroxyden, wie Wasserstoffperoxyd, oder Persäuren, z.B. einer gegebenenfalls durch Niederalkyl, Niederalkoxy, Halogen oder einer weiteren Carboxylgruppe substituierten Benzoepersäure, wie Benzoepersäure selbst oder Phthalmonopersäure, oder einer Alkanpercarbonsäure, wie Peressigsäure oder einem Perjodat, wie Natriumperjodat durchführen. Diese Reaktion wird meist bei tiefen Temperaturen in einem Lösungsmittel, wie Eisessig oder Aceton vorgenommen.

Die neuen Verbindungen können in Form von Säureadditionssalzen, insbesondere pharmazeutisch verwendbaren, nicht-toxischen Salzen, z.B. mit organischen Säuren, wie Chlorwasserstoff-, Bromwasserstoff-, Schwefel- oder Phosphorsäure, oder mit organischen, wie aliphatischen, cycloaliphatischen, cycloaliphatisch-aliphatischen, aromatischen, araliphatischen, heterocyclischen oder heterocyclisch-aliphatischen Carbon- oder Sulfonsäure, z.B.

Essig-, Propion-, Bernstein-, Glykol-, Milch-, Aepfel-, Wein-, Zitronen-, Ascorbin-, Malein-, Phenylessig-, Benzoe-, 4-Aminobenzoe-, Anthranil-, 4-Hydroxybenzoe-, Salicyl-, Aminosalicyl-, Embon- oder Nicotin-, sowie Methansulfon-, Aethansulfon-, 2-Hydroxyäthansulfon-, Aethylensulfon-, Benzolsulfon-, p-Toluolsulfon-, Naphthalinsulfon-, Sulfanil- oder Cyclohexylsulfaminsäure, vorliegen. Salze dieser Art können z.B. durch Behandeln der freien Verbindungen, mit den Säuren oder mit geeigneten Anionenaustauscherharzen erhalten werden.

Infolge der engen Beziehungen zwischen den neuen Verbindungen in freier Form und in Form ihrer Salze sind im Vorausgegangenen und nachfolgend unter den freien Verbindungen oder den Salzen sinn- und zweckmässig gegebenenfalls auch die entsprechenden Salze bzw. freien Verbindungen zu verstehen.

Die neuen Verbindungen können als Isomerengemische, wie Racemate oder Diastereoisomerengemische, oder in Form der reinen Isomeren, wie optisch aktiven Komponenten, vorliegen. Die Auftrennung von erhaltenen Isomerengemischen in die reinen Isomeren kann nach den bekannten Methoden geschehen. Racemate lassen sich z.B. auf Grund physikalisch-chemischer Unterschide, wie z.B. solchen der Löslichkeit, ihrer diastereomeren Salze, oder durch fraktioniertes Kristallisieren aus einem optisch aktiven Lösungsmittel, oder durch Chromatographie, insbesondere Dünnschichtchromatographie, an einem optisch aktiven Trägermaterial, in die optisch aktiven Antipoden auftrennen. Dabei isoliert man vorteilhafterweise das pharmakologisch wirksamere oder weniger toxische reine Isomere, insbesondere den wirksameren oder weniger toxischen aktiven Antipoden.

Die obigen Reaktionen werden in üblicher Weise in An- oder Abwesenheit von Verdünnungs-, Kondensations- und/oder katalytischen Mitteln, falls notwendig, bei erniedrigter oder erhöhter Temperatur, im geschlossenen Gefäss und/oder in einer Inertgasatmosphäre durchgeführt.

Das Verfahren umfasst auch diejenigen Ausführungsformen, wonach als Zwischenprodukte anfallende Verbindungen als Ausgangsstoffe verwendet und die restlichen Verfahrensschritte mit diesen durchgeführt werden, oder das Verfahren auf irgendeiner Stufe abgebrochen wird; ferner können Ausgangsstoffe in Form von Derivaten verwendet oder während der Reaktion gebildet werden.

Vorzugsweise werden solche Ausgangsstoffe verwendet und die Reaktionsbedingungen so gewählt, dass man zu den eingangs als besonders bevorzugt aufgeführten Verbindungen gelangt.

Die neuen Verbindungen der vorliegenden Erfindung können z.B. zur Herstellung von pharmazeutischen Präparaten verwendet werden, welche eine wirksame Menge der Aktivsubstanz zusammen oder im Gemisch mit anorganischen oder organischen, festen oder flüssigen, pharmazeutisch verwendbaren Trägerstoffen enthalten, die sich zur enteralen oder parenteralen Verabreichung eignen. So verwendet man Tabletten oder Gelatinekapseln, welche den Wirkstoff zusammen mit Verdünnungsmitteln, z.B. Laktose, Dextrose, Sukrose, Mannitol, Sorbitol, Cellulose und/oder Glycerin, und Schmiermitteln, z.B. Kieselerde, Talk, Stearinsäure oder Salze davon, wie Magnesium- oder Calciumstearat, und/oder Polyäthylenglykol, aufweisen; Tabletten enthalten ebenfalls Bindemittel, z.B. Magnesiumaluminiumsilikat, Stärken, wie Mais-, Weizen-, Reis- oder Pfeilwurzstärke, Gelatine, Traganth, Methylcellulose, Natriumcarboxymethylcellulose und/oder Polyvinylpyrrolidon, und, wenn erwünscht, Sprengmittel, z.B. Stärken, Agar, Alginsäure oder ein Salz davon, wie Natriumalginat, und/oder Brausemischungen, oder Adsorptionsmittel, Farbstoffe, Geschmackstoffe und Süssmittel. Ferner kann man die neuen pharmakologisch wirksamen Verbindungen in Form von injizierbaren, z.B. intravenös verabreichbaren Präparaten oder von Infusionslösungen verwenden. Solche Lösungen sind vorzugsweise isotonische wässrige Lösungen oder Suspensionen, wobei diese z.B. aus lyophilisierten Präparaten, welche die Wirksubstanz allein oder zusammen mit einem Trägermaterial, z.B. Mannit, enthalten, vor Gebrauch hergestellt werden können. Die pharmazeutischen Präparate können sterilisiert sein und/oder Hilfsstoffe, z.B. Konservier-, Stabilisier-, Netz- und/oder Emulgiermittel, Löslichkeitsvermittler, Salze zur Regulierung des osmotischen Druckes und/oder Puffer enthalten. Die vorliegenden pharmazeutischen Präparate, die, wenn erwünscht, weitere pharmakologisch wertvolle Stoffe enthalten können, werden in an sich bekannter Weise, z.B. mittels konventioneller Misch-, Granulier-, Dragier-, Lösungs- oder Lyophilisierungsverfahren, hergestellt und enthalten von etwa 0,1% bis 100%, insbesondere von etwa 1% bis etwa 50%, Lyophilisate bis zu 100% des Aktivstoffes. Die Einzeldosis für einen Warmblüter von etwa 70 kg Gewicht beträgt zwischen 0,1 und 0,75 g, die Tagesdosis zwischen 0,2 und 1,0 g.

Die folgenden Beispiele dienen zur Illustration der Erfindung; Temperaturen werden in Celsiusgraden angegeben.

Beispiel 1: 45 g Brom-desoxy-anisoin werden in 170 ml Acetonitril suspendiert. Die Suspension wird mit 23 ml N-Aethyldiisopropylamin und mit 15,4 g 2-Aminothiazolin versetzt, 2 Stunden bei Raumtemperatur gerührt, abgenutscht und das Nutschgut mit wenig Essigester nachgewaschen. Man erhält so das 2-Imino-3-(1,2-bis-p-methoxyphenyl-2-oxo-äthyl)-thiazolin vom F. = 95-98°.

In analoger Weise erhält man das 2-Imino-5-methyl-3-(1,2-Bis-p-methoxyphenyl-2-oxo-äthyl)-thiazolin oder seine Tautomeren,

das 2-Imino-4-methyl-3-(1,2-bis-p-methoxyphenyl-2-oxo-äthyl)-thiazolin oder seine Tautomeren,

das 2-Imino-3-(1,2-bis-phenyl-2-oxo-äthyl)-thiazolin oder seine Tautomeren vom F. 121-123°, und

das 2-Imino-3-(1,2-bis-p-methoxyphenyl-2-oxo-äthyl)-5,6-dihydro-4H-thiazin oder seine Tautomeren.

Patentansprüche:

1. Verbindungen der Formel I

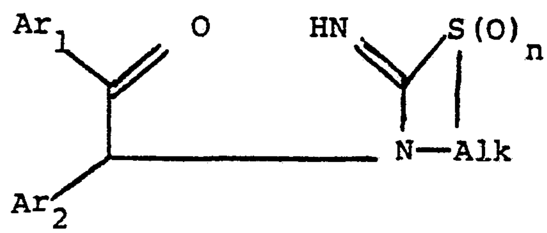

(I)

oder ihre Tautomeren, worin Alk Niederalkylen darstellt, welches das Thia- vom Aza-atom durch 2-4 Kohlenstoffatome trennt, $Ar_1$ und $Ar_2$ unabhängig voneinander gegebenenfalls substituiertes Phenyl, Pyridyl oder Thienyl bedeuten und n O, 1 oder 2 ist, mit der Massgabe, dass $Ar_1$ von Phenyl, Monohalogenphenyl, Mono-$C_1$-$C_4$-Alkoxyphenyl und Mono-$C_1$-$C_6$-Alkylphenyl, $Ar_2$ von Monohalogenphenyl und Mono-$C_1$-$C_4$-Alkoxyphenyl oder n von O verschieden ist, wenn

Alk das Thio- vom Aza-atom durch 2 Kohlenstoffatome trennt, und mit der weiteren Massgabe, dass mindestens einer der Reste $Ar_1$ und $Ar_2$ von gegebenenfalls substituiertem Phenyl verschieden ist, wenn Alk Aethylen ist, und deren Salze.

2. Verbindungen gemäss Anspruch 1 der Formel I oder ihre Tautomeren, worin Alk ein Niederalkylenrest ist, der das Schwefel- und Stickstoffatom über 2-3 Kohlenstoffatome miteinander verbindet, $Ar_1$ und $Ar_2$ unabhängig voneinander einen gegebenenfalls durch Niederalkoxy oder Halogen substituierten Phenylrest und n O, 1 oder 2 bedeutet, und deren Salze.

3. Verbindungen der Formel I

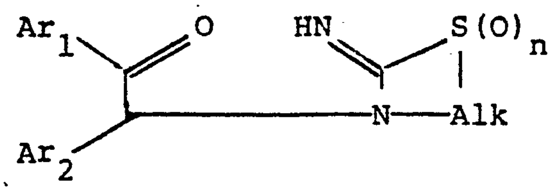 (I)

und ihre Tautomeren, worin $Ar_1$ und $Ar_2$ gegebenenfalls substituiertes Phenyl bedeuten, Alk Aethylen ist und n für O, 1 oder 2 steht, oder worin $Ar_1$ Phenyl, Monohalogenphenyl, Mono-$C_1$-$C_4$-Alkoxyphenyl oder Mono-$C_1$-$C_6$-Alkylphenyl bedeutet, $Ar_2$ Monohalogenphenyl oder Mono-$C_1$-$C_4$-Alkoxyphenyl darstellt, Alk Niederalkylen bedeutet, welches das Thia- vom Aza-atom durch 2 Kohlenstoffatome trennt, und n für O steht, und ihre Salze, frei von an der Reaktion von Verbindungen der Formeln

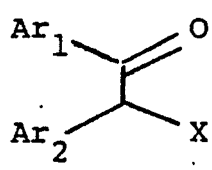 (III) und 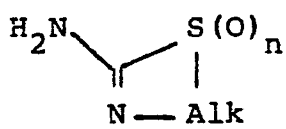 (IV),

worin X Brom ist und $Ar_1$, $Ar_2$, Alk und n obige Bedeutungen haben, teilnehmenden Reagenzien und von ihnen verschiedenen Reaktionsprodukten.

4. 2-Imino-3-(1,2-bis-p-methoxy-phenyl-2-oxo-äthyl)-thiazolin oder seine Tautomeren, frei von den an der Reaktion von α-Bromdesoxyanisoin mit 2-Aminothiazol teilnehmenden Reagenzien und den von ihnen verschiedenen Reaktionsprodukten.

5. 2-Imino-3-(1,2-bis-phenyl-2-oxo-äthyl)-thiazolin oder seine Tautomeren, frei von den an der Reaktion von α-Bromdesoxybenzoin mit 2-Aminothiazol teilnehmenden Reagenzien und von denen verschiedenen Reaktionsprodukten.

6. 2-Imino-3-(1,2-bis-p-methoxy-phenyl-2-oxo-äthyl)-5,6-dihydro-4H-thiazin oder dessen Tautomere.

7. Verbindungen gemäss einem der Ansprüche 1-6 mit antiphlogistischer und/oder analgetischer Wirkung.

8. Pharmazeutische Präparate enthaltend eine Verbindung der Formel I

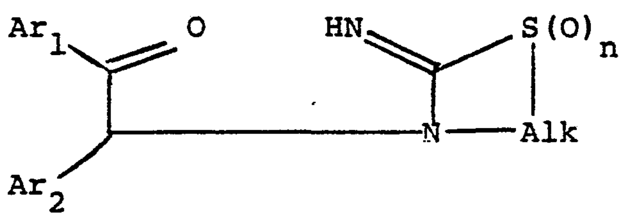

(I)

oder ihre Tautomeren, worin Alk Niederalkylen darstellt, welches das Thia- vom Aza-atom durch 2-4 Kohlenstoffatome trennt, $Ar_1$ und $Ar_2$ unabhängig voneinander gegebenenfalls substituiertes Phenyl, Pyridyl oder Thienyl bedeuten und n 0, 1 oder 2 ist oder ein Salz davon neben üblich pharmazeutischen Hilfs- und Trägerstoffen.

9. Pharmazeutische Präparate gemäss Anspruch 8 enthaltend eine Verbindung mit antiphlogistischer und/oder analgetischer Wirkung neben üblich pharmazeutischen Hilfs- und Trägerstoffen.

10. Verfahren zur Herstellung von 1-Thia-3-aza-cyclopentanen der allgemeinen Formel I

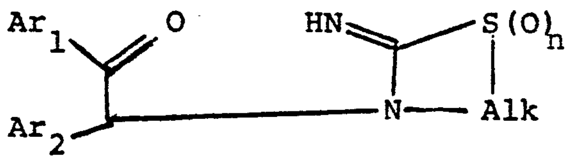

(I)

worin Alk Niederalkylen darstellt, welches das Thia- vom Aza-atom durch 2-4 Kohlenstoffatomen trennt, $Ar_1$ und $Ar_2$ unabhängig voneinander gegebenenfalls substituiertes Phenyl, Pyridyl oder Thienyl bedeuten und n 0, 1 oder 2 ist, mit der Massgabe, dass $Ar_1$ von Phenyl, Monohalogenphenyl, Mono-$C_1$-$C_4$-Alkoxyphenyl und Mono-$C_1$-$C_6$-Alkylphenyl, $Ar_2$ von Monohalogenphenyl und Mono-$C_1$-$C_4$-Alkoxyphenyl oder n von 0 verschieden ist, wenn Alk das Thio- vom Aza-atom durch 2 Kohlenstoffatome trennt, und mit der weiteren Massgabe, dass mindestens einer der Reste $Ar_1$ und $Ar_2$ von gegebenenfalls substituiertem Phenyl verschieden ist, wenn Alk Aethylen darstellt, und deren Tautomeren und/oder Salze, dadurch gekennzeichnet, dass man eine Verbindung der Formel III

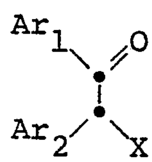

(III)

worin X eine reaktionsfähige veresterte Hydroxygruppe darstellt, mit einer Verbindung der Formel IV

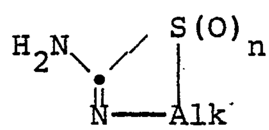

(IV)

oder einem Tautomeren und/oder Salz davon umsetzt, und, wenn erwünscht, in gegebenenfalls erhaltenen Verbindungen, worin n 0 ist, das Thiaatom zur Sulfinyl- oder Sulfonylgruppe oxidiert, und/oder, wenn erwünscht, erhaltene freie

Verbindungen in ihre Salze überführt oder erhaltene Salze in die freien Verbindungen umwandelt und/oder ein verfahrensgemäss erhaltenes Isomerengemisch in die einzelnen Isomeren auftrennt.

11. Verbindungen der Formel I

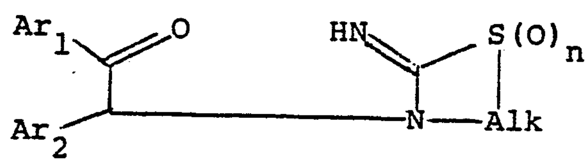

(I)

oder ihre Tautomeren, worin Alk Niederalkylen darstellt, welches das Thia- vom Aza-atom durch 2-4 Kohlenstoffatome trennt, $Ar_1$ und $Ar_2$ unabhängig voneinander gegebenenfalls substituiertes Phenyl, Pyridyl oder Thienyl bedeuten und n 0, 1 oder 2 ist oder deren Tautomere und/oder Salze zur Anwendung in einem Verfahren zur therapeutischen Behandlung des menschlichen oder tierischen Körpers.

12. Verwendung von Verbindungen der Formel I

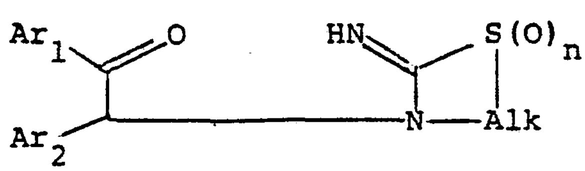

(I)

oder ihre Tautomeren, worin Alk Niederalkylen darstellt, welches das Thia- vom Aza-atom durch 2-4 Kohlenstoffatome trennt, $Ar_1$ und $Ar_2$ unabhängig voneinander gegebenenfalls substituiertes Phenyl, Pyridyl oder Thienyl bedeuten und n 0, 1 oder 2 ist oder deren Tautomere und/oder Salze zur Bekämpfung entzündlicher Erkrankungen.

Europäisches Patentamt

# EUROPÄISCHER RECHERCHENBERICHT

0020858

Nummer der Anmeldung

EP 80 10 1322

| EINSCHLÄGIGE DOKUMENTE | | | KLASSIFIKATION DER ANMELDUNG (Int.Cl. 3) |
|---|---|---|---|
| Kategorie | Kennzeichnung des Dokuments mit Angabe, soweit erforderlich, der maßgeblichen Teile | betrifft Anspruch | |
| A | DE - A - 1 545 569 (AMERICAN CYANAMID)<br>* Seite 6 *<br>-- | 1 | C 07 D 283/02<br>417/06<br>A 61 K 31/425<br>31/54 |
| A | DE - A - 1 900 974 (RHONE-POULENC)<br>* Seiten 1,2 *<br>-- | 1 | |
| A | DE - A - 2 247 822 (ICI)<br>* Seiten 45-49 *<br>---- | 1 | RECHERCHIERTE SACHGEBIETE (Int. Cl. 3)<br>C 07 D 277/18<br>279/06<br>281/02<br>A 61 K 31/425<br>31/54 |

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 06-06-1980 | BRIGHENTI |